# EUROPEAN PATENT SPECIFICATION

(11) **EP 2 335 705 B1**
(45) Date of publication and mention of the grant of the patent: **18.12.2013**
(21) Application number: 11001424.8
(22) Date of filing: 02.08.2007
(51) Int. Cl.: A61K 31/688, A61K 31/661, A61P 31/16, A61K 35/20, A23L 1/30, A23C 9/14, A61K 9/00

(54) **Use of sphingomyelin for the treatment of influenza**
Verwendung von Sphingomyelin zur Behandlung von Influenza
Utilisation de la sphingomyéline pour le traitement du virus de la grippe

(30) Priority: 04.08.2006 JP 2006213273; 04.08.2006 JP 2006213276
(43) Date of publication of application: 22.06.2011
(62) Divisional of application: 07805892.2
(73) Proprietor: MEGMILK SNOW BRAND Co., Ltd., Higashi-ku Sapporo (JP)
(72) Inventor: Kawakami, Hiroshi, Kawagoe-shi Saitama 350-1142 (JP)
(74) Representative: Grünecker, Kinkeldey, Stockmair & Schwanhäusser

(56) References cited:
- WO-A1-2005/097199
- US-A1- 2004 077 541

## Description

### TECHNICAL FIELD

The present invention relates to an agent for preventing infection with an influenza virus, which consists of a sphingosine-containing phospholipid, particularly sphingomyelin, as an active ingredient.

### BACKGROUND OF THE INVENTION

An influenza virus causes prevalence almost annually, by infecting people from the air. Though its threat has been declining in recant years due to an improvement of sanitation and progress in medical science, there still are cases of generating the dead.

There are three types of type A, type B and type C in the influenza virus, and among them, the type A virus is apt to generate mutation and apt to induce a world-wide flu epidemic. Prevention against the infection with an influenza virus is mainly carried out by an inoculation of vaccine. However, since the influenza virus is apt to cause antigenic shift, antigenic drift and the like mutations, the prevalent virus does not coincide with an antigen of the vaccine in many cases, so that it is the present situation that the effect of prevention by vaccine is not satisfactory.

Because of this, preventative inoculation of vaccine to children is also not under obligation now. Amantadine, Oseltamivir and the like are cited as the therapeutic agents for the influenza, but it is necessary to take care of their use because it is necessary to take side effects, development of resistant bacteria and the like problems into consideration. Based on such situations, concern has been directed toward an agent or food or drink which can be ingested daily and safely and is effective in preventing infection with an influenza virus.

### DISCLOSURE OF THE INVENTION

### PROBLEMS THAT THE INVENTION IS TO SOLVE

An object of the invention is to provide an agent for use in preventing infection with an influenza virus, which can be ingested daily and safely.

### MEANS FOR SOLVING THE PROBLEMS

Said object was solved by an agent for use in preventing an infection with an influenza virus, which consists of a sphingosine-containing phospholipid.

That is, the invention relates to the following embodiments. An agent for use in preventing an infection with an influenza virus, which consists of a sphingosine-containing phospholipid as an active ingredient.
The agent for use in preventing an infection with an influenza virus according to the above embodiment, wherein the sphingosine-containing phospholipid is sphingomyelin.
Preferred embodiments of the invention are set forth in the dependent claims.

### ADVANTAGE OF THE INVENTION

The infection with an influenza virus can be prevented by the agent for use in preventing infection with an influenza virus of the invention.

Since the agent for use in preventing infection with an influenza virus of the invention consists of a sphingosine-containing phospholipid, particularly sphingomyelin, as an active ingredient, these can be provided in large amounts relatively inexpensively and also have a characteristic of markedly high safety.

### BEST MODE FOR CARRYING OUT THE INVENTION

### (Sphingosine-containing phospholipid)

In spite of the presence of sphingomyelin in a milk in a large amount of from 20 to 30% by mass in phospholipid, studies on its function are limited to cell levels, and information on its physiological functions in a living body is extremely scarce. Accordingly, its effectiveness as a component of nutrient substances has not been recognized.

Regarding applications of sphingomyelin, antiinflammatory and analgesic external preparations, an agent for improving digestion absorption functions of lipid, an agent for treating intestinal movement function insufficiency diseases (JP-A-5-186330, JP-A-11-269074, JP-A-2003-252765) and the like are known, but nothing has been revealed on its preventive effect on the infection with an influenza virus so that it has not been used for the purpose of preventing infection with an influenza virus.

On the other hand, cases of using sphingomyelin as a lipid component for the purpose of forming liposome by emulsifying a component having an anti-viral action (JP-T-2006-508045) (the term "JP-T" as used herein means a published Japanese translation of a PCT patent application) and an antigen to be used as vaccine (JP-A-5-339169) have been reported, but it is not known that the sphingomyelin itself has anti-influenza virus activity.

In addition, it is conventionally known that ganglioside which is a glycolipid contained in a milk has an anti-influenza activity (JP-A-4-105616), but sialic acid which is a constituent component is important for this action which is a function expressed by a mechanism in which a glycolipid comprising sialic acid competitively inhibits binding of the virus with mucous epithelium. Accordingly, it is evident that it does not remind of a function of sphingomyelin which does not comprise sialic acid.

The sphingosine-containing phospholipid, particularly sphingomyelin, to be used in the invention may be purified or may be used as a sphingomyelin-containing phospholipid.

Though sphingomyelin is richly contained in an animal brain and a milk fat, a milk-derived one is desirable from the viewpoint of carrying out the invention. As the milk-derived sphingomyelin, it is desirable to use a raw milk, whey protein concentrate (WPC) and the like as a material.

As the method for obtaining a sphingomyelin-containing phospholipid fraction from the raw milk, WPC and the like, a method for extracting with ether or acetone (JP-A-3-47192), a method for using a water-soluble fraction containing butter curd or butter serum obtained by heat-melting butter, and the like conventionally known methods can be exemplified. The sphingomyelin content of the fraction obtained by employing these materials and methods is about 28% by mass and about 9% by mass, respectively.

In addition, sphingomyelin having increased purity can be obtained by purifying the aforementioned sphingomyelin-containing phospholipid fraction by dialysis, ammonium sulfate fractionation, gel filtration, isoelectric precipitation, ion exchange chromatography, solvent fractionation, ultrafiltration (UF), microfiltration (MF) and the like techniques.

These sphingomyelin and sphingomyelin-containing phospholipid can optionally take a liquid, a powder, a tablet and the like forms and can be orally administered directly. In addition, not only sphingomyelin but also a phospholipid composition containing an effective amount of phosphatidylcholine defined by the human nutrition requirements may be used.

Regarding the blending amount of the sphingosine-containing phospholipid in the agent for use in preventing infection with an influenza virus of the invention, in the case of adults, the blending amount may be adjusted in such a manner that the sphingosine-containing phospholipid, particularly sphingomyelin, can be ingested in an amount of approximately from 0.1 mg to 5000 mg per day. By setting within this range, the preventive action on the infection with an influenza virus can be exerted. Since the sphingosine-containing phospholipid, particularly sphingomyelin, which is the active ingredient of the agent for use in preventing infection with an influenza virus of the invention is a milk component, it can be said that there is no problem regarding its safety even when ingested in a large amount.

As the dosage form of the agent for use in preventing infection with an influenza virus of the invention, for example, tablets, capsules, granules, powdered materials, powders, solutions and the like can be exemplified. These may be orally administered or may be transnasally administered. In addition, these dosage forms can be produced by conventionally known general methods. For example, they are formed by mixing with pharmaceutical preparation production-acceptable carriers, fillers and the like.

The following describes the invention further in detail with reference to examples and test examples.

### Example 1

A reaction liquid obtained by allowing a protease to act upon a 10% by mass aqueous solution of a whey protein concentrate (WPC) was extracted with a chloroform-methanol (2:1) solution, concentrated and further extracted with acetone to obtain a complex lipid fraction. Next, this complex lipid fraction was treated with a fluorosilyl column chromatography and extracted stepwise with chloroform-methanol solutions to obtain a phospholipid fraction. This phospholipid fraction was treated with a silica gel chromatography and extracted stepwise with chloroform-methanol solutions, and the result was freeze-dried to obtain sphingomyelin. This sphingomyelin was treated with a thin layer chromatography and then color-developed with Dittmer's reagent and measured by a densitometry to find that the sphingomyelin content was 95.2% by mass. It is possible to use the sphingomyelin obtained in this manner directly as an agent for preventing infection with an influenza virus.

### [Test Example 1]

### (Verification of the effect to prevent infection with type A influenza virus and type B influenza virus)

Mice (Balb/c, male, 6 weeks of age) were transnasally infected with A/Guinzhou virus as the type A influenza virus or B/Ibaraki virus as the type B influenza virus, and at the same time, a 100 µg/ml solution of the sphingomyelin (SPM) obtained in Example 1 was transnasally administered at a dose of 5 µl/nasal cavity dose (dose: 0.5 ug), and the preventive effect on the infection with an influenza virus was judged by the virus titer in the nasal cavity washes. In this connection, groups transnasally infected with respective influenza viruses alone were prepared. A plaque method which uses MDCK cell was used in the judgment.

The results are shown in Tablet 1. As a result of this, infection preventive effect by the administration of sphingomyelin was confirmed for both of the type A and type B. Particularly, more considerable effect was confirmed for the type A influenza virus.

**[Table 1]**

| | Sample | Nasal cavity virus titer |
|---|---|---|
| A/Guizhou | Control | 2.93 ± 0.08 |
| virus | SPM | 1.99 ± 0.31 |
| | | |
| B/Ibaraki | Control | 2.91 ± 0.12 |
| virus | SPM | 2.73 ± 0.06 |

### [Test Example 2]

### (Verification of the effect of oral administration of sphingomyelin on the prevention of infection with an influenza virus)

A mouse (Balb/c, male, 6 weeks of age) was infected with 1 x 10³ pfu in viral quantity of influenza virus PR 8 (H1N1). Before the infection treatment, sphingomyelin was orally administered, and its infection preventive effect was judged by the virus titer in the nasal cavity washes 3 days after the virus infection. In carrying out the oral administration, sphingomyelin was used by dispersing in water. A plaque method which uses MDCK cell was used in the judgment.

The results are shown in Table 2. The nasal cavity virus titer was lowered with significance in the sphingomyelin administration group. This indicates the effect of sphingomyelin to prevent infection with an influenza virus.

**[Table 2]**

| Sample | | Nasal cavity virus titer x (PFU/ml: 10^{x}) |
|---|---|---|
| Control (no administration) | | 2.91 ± 0.07 |
| | | |
| Sphingomyelin administration | 1 mg | 2.23 ± 0.16 |
| Sphingomyelin administration | 10 mg | 2.15 ± 0.29 |

### Example 2

Solutions for intra-nasal cavity spray were produced by dissolving 5 g of the sphingomyelin obtained in Example 1 in 200 ml of distilled water for injection.

### Example 3

The materials of the formulation shown in Table 3 were mixed, made into granules and then filled in capsules, thereby producing capsules for preventing infection with an influenza virus.

**[Table 3]**

| | | |
|---|---|---|
| Sphingomyelin (Example 7) | 20.0 | (% by mass) |
| Lactose | 24.5 | |
| Soluble starch | 55.0 | |
| Magnesium stearate | 0.5 | |

### Example 4 (Reference)

The materials of the formulation shown in Table 4 were mixed, filled in a container and then heat-sterilized, thereby producing a food or drink for preventing infection with an influenza virus.

**[Table 4]**

| | | |
|---|---|---|
| Sphingomyelin (Example 7) | 2.5 | (% by mass) |
| Sucrose | 7.5 | |
| Citric acid | 0.6 | |
| Apple juice | 10.0 | |
| Water | 79.4 | |

## Claims

1. An agent for use in preventing an infection with an influenza virus, which consists of a sphingosine-containing phospholipid.

2. The agent for use according to claim 1, wherein the amount of the agent is such that it can be ingested in an amount of 0.1 mg to 5000 mg per day.

3. The agent for use according to claim 1 or 2, wherein the sphingosine-containing phospholipid is sphingomyelin.

## Patentansprüche

1. Mittel zur Verwendung beim Verhüten einer Infektion mit einem Influenzavirus, welches aus einem Sphingosin enthaltenden Phospholipid besteht.

2. Mittel zur Verwendung nach Anspruch 1, wobei die Menge des Mittels so ist, dass es in einer Menge von 0,1 mg bis 5000 mg pro Tag eingenommen werden kann.

3. Mittel zur Verwendung nach Anspruch 1 oder 2, wobei das Sphingosin enthaltende Phospholipid Sphingomyelin ist.

## Revendications

1. Agent destiné à être utilisé pour prévenir une infection par un virus grippal, ledit agent étant constitué d'un phospholipide contenant de la sphingosine.

2. Agent destiné à être utilisé selon la revendication 1, où la quantité de l'agent est telle qu'il peut être ingéré en une quantité de 0,1 mg à 5000 mg par jour.

3. Agent destiné à être utilisé selon la revendication 1 ou 2, où le phospholipide contenant de la sphingosine est la sphingomyéline.
